# EUROPEAN PATENT APPLICATION

(11) **EP 3 351 532 A1**
(43) Date of publication of application: **25.07.2018**
(21) Application number: 18152522.1
(22) Date of filing: 19.01.2018
(51) Int. Cl.: C07D 211/58

(54) **METHOD FOR PREPARING PIMAVANSERIN**

(30) Priority: 20.01.2017 US 201762448712 P
(71) Applicant: SCI Pharmatech, Inc., Taoyuan City 338 (TW)
(72) Inventor: HUANG, Chen-Wei, 338 Taoyuan City (TW); TSENG, Chin-Wei, 338 Taoyuan City (TW)
(74) Representative: Epping - Hermann - Fischer

(57) **Abstract**

Provided is a method for preparing pimavanserin including reacting an intermediate compound represented by Formula (II) with N-(4-fluorobenzyl)-1-methylpiperidin-4-amine or a salt thereof, or reacting an intermediate compound represented by Formula (IV) with 4-isobutoxybenzylamine or a salt thereof, wherein L represents a heteroaryl group, -OR₁ or halogen, and wherein R₁ represents C₁ to C₁₀ alkyl or aryl. The present disclosure provides the method for preparing pimavanserin without the use of isocyanate intermediate.

## Description

### BACKGROUND

### 1. Technical Field:

The present disclosure relates to a method for preparing pimavanserin, especially to a method for preparing pimavanserin without the use of isocyanate intermediate.

### 2. Description of Associated Art:

Pimavanserin, i.e., 1-(4-fluorobenzyl)-3-(4-isobutoxybenzyl)-1-(1-methyl-piperidin- 4-yl)-urea, is a 5-HT_{2A} modulator used for Parkinson's disease associated with psychosis. It was developed by Acadia Pharmaceuticals. The method for preparation of pimavanserin by using the step of reaction of an isocyanate intermediate with a secondary amine has been described in U.S. Patent No. 8,236,960 and U.S. Patent Application Publication No. 2015/0313888. However, the isocyanate intermediate was prepared from a highly toxic phosgene reaction with 4-isobutoxybenzylamine hydrochloride. Therefore, it should be very careful to handle it in use.

Accordingly, it is an urgent and important issue to provide a method for preparation of pimavanserin without the use of the toxic isocyanate intermediate.

### SUMMARY

In view of the foregoing, the present disclosure provides a method for preparing pimavanserin, comprising reacting an intermediate compound represented by Formula (II) with N-(4-fluorobenzyl)-1-methylpiperidin-4-amine represented by Formula (III) or a salt thereof, or reacting an intermediate compound represented by Formula (IV) with 4-isobutoxybenzylamine represented by Formula (V) or a salt thereof, wherein L represents a heteroaryl group, -OR₁ or halogen, and wherein R₁ represents C₁ to C₁₀ alkyl or aryl.

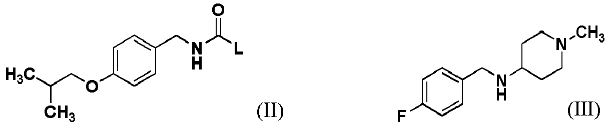

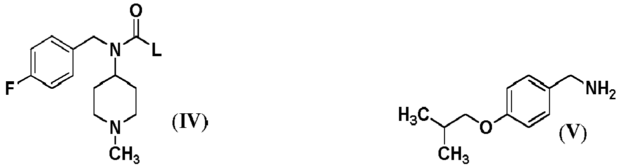

### DETAILED DESCRIPTION OF THE EMBODIMENTS

The following specific examples are used to exemplify the present disclosure. A person of ordinary skills in the art can conceive the other advantages of the present disclosure, based on the disclosure of the specification of the present disclosure. The present disclosure can also be implemented or applied as described in different specific examples.

The present disclosure provides a method for preparing pimavanserin, which may include reacting an intermediate compound represented by Formula (II) with N-(4-fluorobenzyl)-1-methylpiperidin-4-amine represented by Formula (III) or a salt thereof, or reacting an intermediate compound represented by Formula (IV) with 4-isobutoxybenzylamine represented by Formula (V) or a salt thereof. In the Formulas (II) and (IV), L represents a heteroaryl group, -OR₁ or halogen, and R₁ represents C₁ to C₁₀ alkyl or aryl.

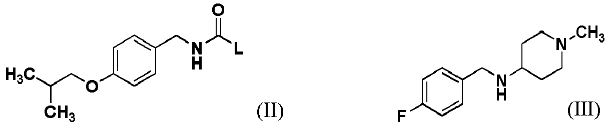

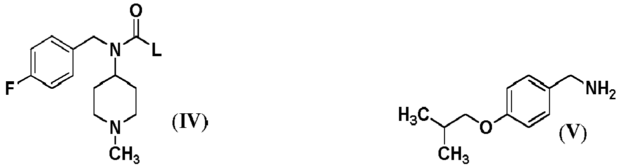

In an embodiment of the present disclosure, the heteroaryl group may be imidazole.

In an embodiment of the present disclosure, the intermediate compound represented by Formula (II) may be obtained by reacting 4-isobutoxybenzylamine represented by Formula (V) or a salt thereof with carbonyl diimidazole.

In an embodiment of the present disclosure, the salt of 4-isobutoxybenzylamine may be hydrochloride, acetate, sulfate or phosphate. In another embodiment, the salt of 4-isobutoxybenzylamine is hydrochloride.

In an embodiment of the present disclosure, 4-isobutoxybenzylamine represented by Formula (V) is obtained by reduction of 4-isobutoxybenonitrile through a hydrogenation or a hydride reduction reaction shown as follows:

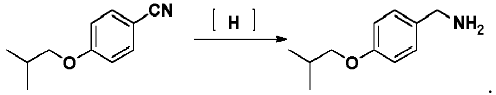

In an embodiment of the present disclosure, the hydrogenation reaction may be carried out in the presence of a catalyst. In another embodiment of the present disclosure, the hydrogenation reaction may be carried out under a neutral or acidic condition. In another embodiment of the present disclosure, the hydrogenation reaction may be carried out under a neutral or acidic condition in the presence of a catalyst. In one embodiment, the catalyst is Pd/C, Pt, Raney Ni or Ni. In another embodiment, the catalyst is 5% Pd/C or 10% Pd/C.

In an embodiment of the present disclosure, the hydride reduction reaction may be carried out by reaction with a hydride reagent. In one embodiment, the hydride reagent is sodium bis (2-methoxyethoxy) aluminium hydride, LiAlH₄ or NaBH₄. In another embodiment, the hydride reagent is LiAlH₄.

In an embodiment of the present disclosure, the method may be carried out in the presence of an aprotic polar solvent or a non-polar solvent. In one embodiment, the aprotic polar solvent or the non-polar solvent may be chosen from acetonitrile, dimethylformamide, dimethylsulfoxide, ethyl acetate, dichloromethane, tetrahydrofuran, toluene and heptanes. In another embodiment, the aprotic polar solvent or the non-polar solvent may be acetonitrile or dimethylformamide.

In an embodiment of the present disclosure, the intermediate compound represented by Formula (IV) may be obtained by reacting N-(4-fluorobenzyl)-1-methylpiperidin-4-amine represented by Formula (III) or a salt thereof with carbonyl diimidazole.

In an embodiment of the present disclosure, the salt of N-(4-fluorobenzyl)-1-methylpiperidin-4-amine represented by Formula (III) is hydrochloride, acetate, sulfate or phosphate. In another embodiment, the salt of N-(4-fluorobenzyl)-1-methylpiperidin-4-amine is hydrochloride.

In an embodiment of the present disclosure, the reacting 4-isobutoxybenzylamine represented by Formula (V), N-(4-fluorobenzyl)-1-methylpiperidin-4-amine represented by Formula (III), or a salt thereof with carbonyl diimidazole is carried out in the presence of a non-alcoholic solvent or a non-polar solvent. In one embodiment, the non-alcoholic solvent or the non-polar solvent may be chosen from acetonitrile, dimethylformamide, dimethylsulfoxide, ethyl acetate, dichloromethane, tetrahydrofuran, toluene and heptanes. In another embodiment, the non-alcoholic solvent or the non-polar solvent may be acetonitrile or dimethylformamide.

In an embodiment of the present disclosure, the method may be carried out at a temperature between an ambient temperature and 100°C. In another embodiment, the method may be carried out at a temperature between an ambient temperature and 70°C.

In an embodiment of the present disclosure, the method for preparing pimavanserin represented by formula (I) could be demonstrated by the following schemes: 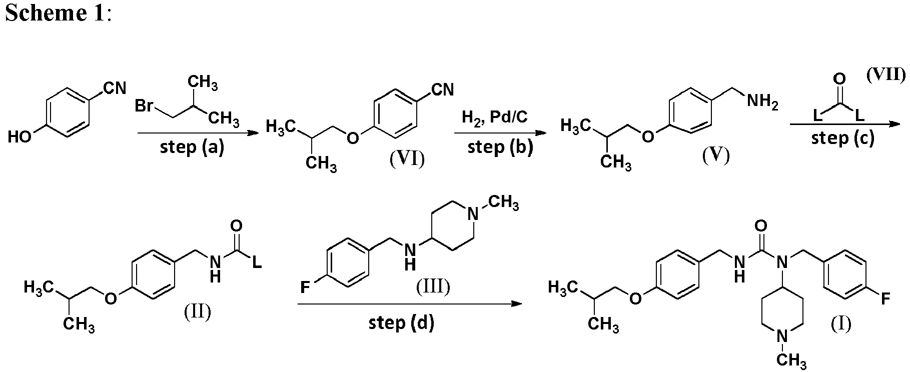 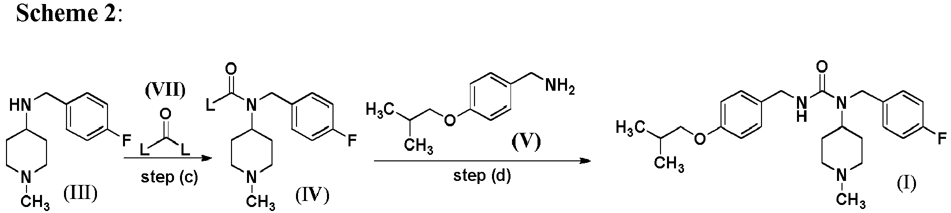 wherein L represents a heteroaryl group, -OR₁ or halogen, and R₁ represents C₁ to C₁₀ alkyl or aryl.

Schemes 1 and 2 are described in detail by the following steps:
Step (a) Alkylation of 4-hydroxybenzonitrile to form 4-isobutoxybenzonitrile represented by formula (VI) 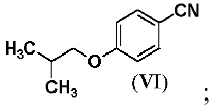
Step (b) Reduction of 4-isobutoxybenzonitrile represented by formula (VI) to form (4-isobutoxyphenyl)methanamine represented by formula (V) 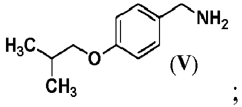
Step (c) Acylation of amine represented by formula (V) or (III) to form an intermediate represented by formula (II) or (IV), respectively 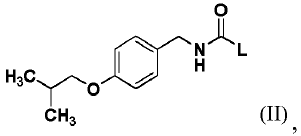 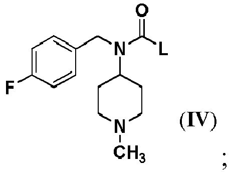
Step (d) Nucleophilic substitution of the intermediate represented by formula (II) or (IV) by amine represented by formula (III) or (V), respectively, to afford pimavanserine represented by formula (I)

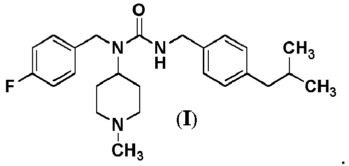

Step (a) in this process is alkylation of 4-hydroxybenzonitrile with alkyl halide in the presence of weak base, protic solvent for forming 4-isobutoxybenzonitrile represented by formula (VI). The alkyl halide may be chosen from chloride, bromide and iodide having isobutyl moiety. Preferably, the alkyl halide is isobutyl bromide. The weak basic, protic solvent may be chosen from sodium bicarbonate, potassium carbonate and tertiary amine, such as triethyl amine, diisopropylethyl amine and N-methyl morpholine. Preferably, the weak basic, protic solvent is potassium carbonate.

Step (b) in this process is reduction of 4-isobutoxybenzonitrile represented by formula (VI) for forming (4-isobutoxyphenyl)methanamine represented by formula (V). This reaction can be carried out in various ways. For example, Step (b) may be carried out by reaction with a hydride reagent such as aluminum hydrides and boron hydrides, or by hydrogenation in the presence of metal as a catalyst such as Pd/C, Pt, Raney Ni, Ni, Pt, and Rh. Preferably, Step (b) is carried out by hydrogenation.

Step (c) in this process is acylation of amine represented by formula (III) or (V) for forming an intermediate represented by formula (IV) or (II), respectively. Particularly, the intermediate represented by formula (IV) can be obtained from acylation of amine represented by formula (III), while the intermediate represented by formula (II) can be obtained from acylation of amine represented by formula (V). The agent used in Step (c) may be a compound represented by formula (VII), such as carbonyldiimidazole (CDI), C₁-C₁₀ alkyl chloroformate and C₁-C₁₀ alkyl carbonate. Preferably, the agent used in Step (c) is carbonyldiimidazole. The operating temperature may range from 0°C to 20°C, and is preferably lower than 10°C, more preferably from 0°C to 5°C.

Step (d) in this process is nucleophilic substitution of an intermediate represented by formula (II) or (IV) by amine represented by formula (III) or (V) for forming pimavanserine represented by formula (I). Particularly, pimavanserine can be obtained from nucleophilic substitution of an intermediate represented by formula (II) by amine represented by formula (III), or from nucleophilic substitution of an intermediate represented by formula (IV) by amine represented by formula (V).

The following are specific embodiments further demonstrating the efficacy of the current disclosure, but not to limit the scope of the current disclosure.

### EXAMPLE

### Example 1: Preparation of N-(4-fluorobenzyl)-1-methylpiperidin-4-amine (Formula (III)) and its hydrochloride salt

30.1 g of 4-fluorobenzyl amine (0.24 moles) and 28.7 g of N-methyl-4-piperidinone (0.25 moles) were dissolved in 151.7 g of MeOH in the presence of 5% Pd/C catalyst (1.1 g) under H₂ until no more H₂ was consumed. The reaction mixture was then filtered and washed with MeOH. The filtrate was concentrated and stripped with ethylacetate (EA) to give oil residue A (50.9 g). 1.11g of the oil residue A (0.05 mole) was dissolved in isopropyl alcohol and then slowly treated with 32% HCl (11.4 g, 0.1 mole). After then, the solid was precipitated out and filtered at 0°C to 5°C to obtain the cake of N-(4-fluorobenzyl)-1-methylpiperidin-4-amine and its hydrochloride salt. The cake was analyzed by UPLC, and the result showed that the purity of N-(4-fluorobenzyl)-1-methylpiperidin-4-amine and its hydrochloride salt was 99%.

### Example 2: Preparation of 4-isobutoxybenzonitrile (Formula (VI))

205.5 g of isobutyl bromide (1.5 moles) was added to 200 g of dimethylformamide (DMF) comprising 119.1 g of 4-hydroxybenzonitrile (1.0 mole) and 300 g of potassium carbonate (2.2 moles) under stirring, and the temperature was raised to 70°C for overnight reaction. Subsequently, 5% KOH (100 g), 400 g of H₂O and 250 g of ethyl acetate were added to the reaction mixture. The organic layer was collected and washed with 1050 g of distilled water for 4 times, followed by concentrated to obtain 178.1 g of 4-isobutoxybenzonitrile in an oil residue form. 4-Isobutoxybenzonitrile was analyzed by UPLC and the result showed that the purity of 4-isobutoxybenzonitrile was 97%.

### Example 3: Preparation of (4-isobutoxyphenyl) methanamine (Formula (V))

10.1 g of 4-isobutoxybenzonitrile (0.06 moles) as prepared in Example 2 were dissolved in 50 g of ethanol and 3.6 g of HOAc (0.06 mole) in the presence of 5% Pd/C catalyst (1.0 g) under H₂ until no more H₂ was consumed. The reaction mixture was filtered and washed with ethanol. The filtrate was then basified with 2% KOH (105 g) to reach a pH value of 9 and impurities were extracted with 70 g of EA. The aqueous phase was concentrated to obtain 6.3 g of (4-isobutoxyphenyl) methanamine in an acetate form. (4-Isobutoxyphenyl) methanamine was analyzed by UPLC, and the result showed that the purity of (4-isobutoxyphenyl) methanamine was 98%.

### Example 4: Preparation of 1-(4-fluorobenzyl)-3-(4-isobutoxybenzyl)-1-(1-methyl piperidin-4-yl) urea (pimavanserin) from (4-isobutoxyphenyl) methanamine acetate

6.3g of (4-isobutoxyphenyl) methanamine acetate (0.03 moles) as prepared in Example 3 was added to EA comprising 8.3 g of carbonyldiimidazole (0.05 moles) under stirring at ambient temperature. Until no (4-isobutoxyphenyl) methanamine acetate was detected, 5.3 g of N-(4-fluorobenzyl)-1-methylpiperidin-4-amine (0.02 moles) as prepared in Example 1 was added and the temperature was raised to 50°C. 150 g of distilled water was then added to quench and 25 g of EA was added to extract the product. Organic layer was washed with 100 g of distilled water and then concentrated to obtain 6.2 g of 1-(4-fluorobenzyl)-3-(4-isobutoxybenzyl)-1-(1-methyl piperidin-4-yl) urea in a light-yellow solid form. 1-(4-Fluorobenzyl)-3-(4-isobutoxybenzyl)-1-(1-methyl piperidin-4-yl) urea was analyzed by UPLC and the result showed that the purity of 1-(4-fluorobenzyl)-3-(4-isobutoxybenzyl)-1-(1-methyl piperidin-4-yl) urea was 90%.

### Example 5: Preparation of 1-(4-fluorobenzyl)-3-(4-isobutoxybenzyl)-1-(1-methyl piperidin-4-yl) urea (pimavanserin) from (4-isobutoxyphenyl) methanamine hydrochloride

8.6 g of (4-isobutoxyphenyl) methanamine hydrochloride (0.04 moles) was added to 43 g of EA comprising carbonyldiimidazole (0.08 moles) under stirring at ambient temperature. Until no (4-isobutoxyphenyl) methanamine hydrochloride was detected, 9.3 g of N-(4-fluorobenzyl)-1-methylpiperidin-4-amine (0.04 moles) as prepared in Example 1 was added and the temperature was raised to 50°C. 150 g of distilled water was added to quench and 25 g of EA was added to extract the product. Organic layer was washed with 100 g of distilled water and then concentrated to obtain 6.2 g of 1-(4-fluorobenzyl)-3-(4-isobutoxybenzyl)-1-(1-methyl piperidin-4-yl) urea in a light-yellow solid form. 1-(4-fluorobenzyl)-3-(4-isobutoxybenzyl)-1-(1-methyl piperidin-4-yl) urea was analyzed by UPLC and the result was shown in FIG. 5 indicating that the purity of 1-(4-fluorobenzyl)-3-(4-isobutoxybenzyl)-1-(1-methyl piperidin-4-yl) urea in the reaction mixture was 94%.

The disclosure has been described using exemplary preferred embodiments. However, it is to be understood that the scope of the disclosure is not limited to the disclosed embodiments. On the contrary, it is intended to cover various modifications and similar rearrangement. The scope of the claims therefore should be accorded the broadest interpretation so as to encompass all such modifications and similar arrangements.

## Claims

1. A method for preparing pimavanserin, comprising:
reacting an intermediate compound represented by Formula (II), 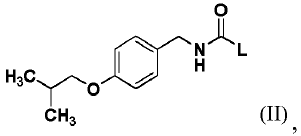 with N-(4-fluorobenzyl)-1-methylpiperidin-4-amine represented by Formula (III) or a salt thereof, 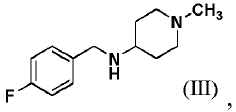 or
reacting an intermediate compound represented by Formula (IV), 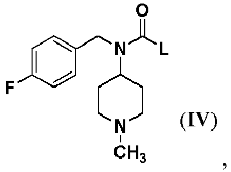 with 4-isobutoxybenzylamine represented by Formula (V) or a salt thereof, 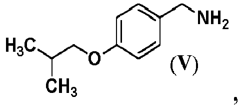
wherein L represents a heteroaryl group, -OR₁ or halogen, and wherein R₁ represents C₁ to C₁₀ alkyl or aryl.

2. The method of claim 1, wherein the heteroaryl group is imidazole.

3. The method of claim 1, further comprising preparing the intermediate compound represented by Formula (II) by reacting 4-isobutoxybenzylamine represented by Formula (V) or a salt thereof with carbonyl diimidazole.

4. The method of claim 3, wherein the salt of 4-isobutoxybenzylamine represented by Formula (V) is hydrochloride, acetate, sulfate or phosphate.

5. The method of claim 3, further comprising preparing 4-isobutoxybenzylamine represented by Formula (V) by reduction of 4-isobutoxybenonitrile through a hydrogenation or a hydride reduction reaction shown as follows:

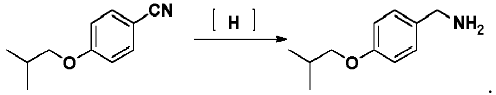

6. The method of claim 5, wherein the hydrogenation reaction is carried out in the presence of a catalyst.

7. The method of claim 6, wherein the catalyst is Pd/C, Pt, Raney Ni or Ni.

8. The method of claim 7, wherein the Pd/C catalyst is 5% Pd/C or 10% Pd/C.

9. The method of claim 5, wherein the hydride reduction reaction is carried out by reaction with a hydride reagent.

10. The method of claim 9, wherein the hydride reagent is sodium bis (2-methoxyethoxy) aluminium hydride, LiAlH₄ or NaBH₄.

11. The method of claim 1, which is carried out in the presence of an aprotic polar solvent or a non-polar solvent.

12. The method of claim 11, wherein the aprotic polar solvent or the non-polar solvent is acetonitrile, dimethylformamide, dimethylsulfoxide, ethyl acetate, dichloromethane, tetrahydrofuran, toluene or heptane.

13. The method of claim 1, further comprising preparing the intermediate compound represented by Formula (IV) by reacting N-(4-fluorobenzyl)-1-methylpiperidin-4-amine represented by Formula (III) or a salt thereof with carbonyl diimidazole.

14. The method of claim 13, wherein the salt of N-(4-fluorobenzyl)-1-methylpiperidin-4-amine represented by Formula (III) is hydrochloride, acetate, sulfate or phosphate.

15. The method of claim 1, which is carried out at a temperature between an ambient temperature and 100°C.
